# EUROPEAN PATENT APPLICATION

(11) **EP 2 760 073 A1**
(43) Date of publication of application: **30.07.2014**
(21) Application number: 12832826.7
(22) Date of filing: 16.01.2012
(51) Int. Cl.: H01M 8/16, H01M 8/02, G01N 27/327, C08K 5/56, B82B 3/00

(54) **ELECTRODE INCLUDING A SELF-ASSEMBLING POLYMER HAVING AN ORGANOMETAL, AND METHOD FOR MANUFACTURING SAME**

(30) Priority: 23.09.2011 KR 20110096141
(71) Applicant: Postech Academy-Industry Foundation, Pohang-si, Gyeongsangbuk-do 790-784 (KR)
(72) Inventor: PARK, Moon Jeong, Pohang-si Gyungbuk790-751 (KR); LEE, Joung Phil, Incheon 403-761 (KR)
(74) Representative: Ahner, Philippe
(86) International application number: PCT/KR2012/000358
(87) International publication number: WO 2013/042835

(57) **Abstract**

The present invention relates to a bioelectrode including a cross-linkable organometallic polymer, and to a method for manufacturing same, and more particularly, to an electrode in which a nanostructure of the organometallic polymer is controlled to be used in bio fuel cells, biosensors, and the like. The electrode according to the present invention includes an organometal and further includes a self-assembling block copolymer and enzyme, and provides usages in bio fuel cells and biosensors.

## Description

### Technical Field

The present invention relates to a bio-electrode comprising a cross-linkable organometallic polymer and a method for fabricating the same. More particularly, the present invention relates to an electrode for use in biofuel cells and biosensors, comprising a nanostructured organometallic polymer.

### Background Art

The development of efficient enzymatic biofuel cells has been arising as a subject of a considerable number of studies in past decades for potential use in such applications as biomedical devices, microchip system, and portable electronics.

Biofuel cells, which are designed to produce electrical power upon consuming ranges of biomass such as alcohols and glucose, have attracted intensive attention due to their environmentally friendly and renewable nature. However, biofuel cells suffer from the disadvantage of being of low power density compared to other energy sources.

This limitation is attributed mostly to the fact that redox active sites are buried within enzyme structures due to enzyme stability, leading to poor interplay between redox reactions and electron transfer. This limitation is also a hindrance to the development of power systems for miniaturized biomedical devices.

Various approaches to enhance the power density of biofuel cells are proposed. Direct electron transfer, which is one of the most widely studied methodologies, is configured to adjust the enzymatic active sites within the electron tunneling distance of an electrode surface. For this, extensive attention has been paid to the use of electron mediators to help electron transfer in these systems by shuttling electrons between enzyme catalytic centers and the current collector. As a result, various materials possessing the ability of electron mediation have been extensively reported in recent years. Examples of such materials include nano-carbons, redox active polymers, cofactor relays, and metal nanoparticles.

To attain more stable and higher current density of biofuel cells in the presence of electron mediators, the essential requirement is the immobilization of enzymes to the electrodes. Consequently, significant amounts of studies on the electron mediators are concerned with the electrical contact of enzymes to the electrode surfaces with covalently attached mediators.

From a wiring perspective, carbon nanotubes have been received great interests thanks to their large specific surface area and excellent electrochemical properties. One drawback of applying carbon nanotubes to the large area electrodes is the difficulty in preventing carbon nanotube aggregations if chemical modification of the carbon nanotubes and/or pre-organization of carbon nanotube arrays are not carried out. In this regard, the concentration of redox sites, redox potentials, and the functionalization of many chemical linker and redox polymers play an important role in controlling electron transfer rates.

PCT/JP2003/009480, issued to SOMY, discloses the fabrication of optimal fuel batteries using various enzymes and enzyme complexes.

However, research into the enhancement of electron mediating properties by morphologically controlling redox polymers still remains insufficient. There is therefore a need for redox polymers of novel, more effective and stable structures.

### Disclosure

### Technical Problem

It is an object of the present invention to provide a novel redox polymer structure possessing a high current density and stability.

It is another object of the present invention to provide a novel electrode structure, composed of an enzyme and a redox polymer, possessing a high current density and stability.

It is a further object of the present invention to provide a method for controlling the morphology of a redox polymer.

It is a still further object of the present invention to provide a novel biofuel cell.

### Technical Solution

In accordance with an aspect thereof, the present invention addresses an electrode comprising a self-assembling organometallic block copolymer and an enzyme.

In accordance with an aspect thereof, the present invention addresses a fuel cell comprising an electrode composed of a self-assembling organometallic block copolymer and an enzyme.

According to one embodiment of the present invention, the enzyme generates an electron using biomass and the electron is transferred through the organic metal. Various known enzymes suitable for the mechanisms of electron generation may be employed. Preferably, an optimal choice is made depending on the fuel used. Examples of the enzymes include glucose dehydrogenase, a series of enzymes in the electron transport system, ATP synthetase, enzymes involved in sugar metabolisms (e.g., hexokinase, glucose phosphate isomerase, phosphofructokinase, fructose bisphosphate aldolase, triose phosphate isomerase glyceraldehyde phosphate dehydrogenase, phosphoglyceromutase, phosphopyruvate hydratase, pyruvate kinase, L-lactate dehydrogenase, D-lactate dehydrogenase, pyruvate dehydrogenase, citrate synthase, aconitase, isocitrate dehydrogenase, 2-oxoglutarate dehydrogenase, succinyl-CoA synthetase, succinate dehydrogenase, malonase fumarase, malonate dehydrogenase, etc.). For glucose, glucose oxidase may be used in combination.

As a fuel useful in the fuel cell of the present invention, sugars such as glucose, alcohols such as ethanol, lipids, proteins, organic acids including intermediates of carbohydrate metabolisms (glucose-6-phosphate, fructose-6-phosphate, fructose-1,6-bisphosphate, triose phosphate isomerase, 1,3-bisphosphoglycerate, 3-phosphoglycerate, 2-phosphoglycerate, phosphoenolpyruvate, pyruvate, acetyl-CoA, citrate, cis-aconitate, isocitrate, oxalosuccinate, 2-oxoglutarate, succinyl-CoA, succinate, fumarate L-malate, oxaloacetate, etc.), or a mixture thereof may be taken. Preferable among them are glucose, ethanol, and intermediates of carbohydrate metabolisms. Particularly preferred is glucose because it is very easy to handle.

No limitations are burdened on the organic metal if it is conductive enough to transfer the electron generated by the enzyme.

Turning to the block copolymer used in the present invention, it is, at least in part, crosslinked, and preferably comprises a cross-linkable block. The cross-linkable block may contain at least one unsaturated group such as a double bond, and may be crosslinked by a metal such as osmium (Os).

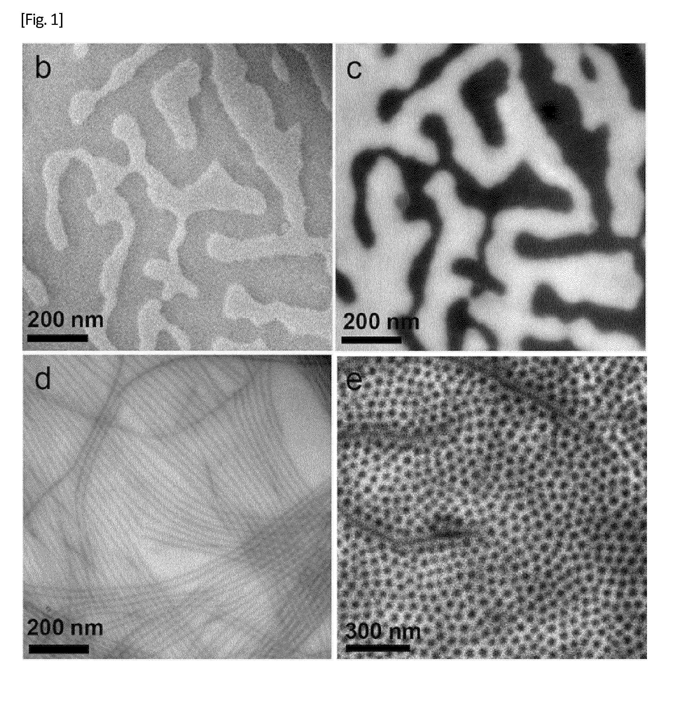

In one embodiment of the present invention, the organometallic block copolymer serving as an electron mediator may be self-assembled to different morphologies including bicontinuous structures, nanowires, and nanoparticles. Preferred is an amorphous bicontinuous structure in which there is a large contact between the crosslinked block copolymer and the enzyme incorporated thereto. The amorphous bicontinuous structure is of the morphologies given in FIG. 1 wherein several metallic domains are connected to each other via bands.

The morphology of the self-assembled block copolymer may vary depending on molecular weight, the kinds of solvent, and compatibility with the solvent.

In one preferred embodiment of the present invention, the block copolymer may be represented by the following General Formula: 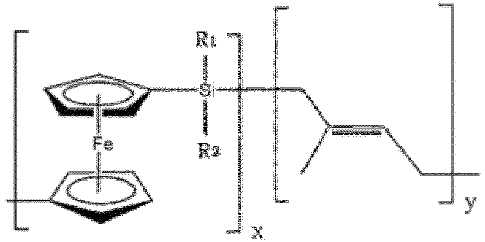 wherein, x and y are the degree of polymerization for each block, and R₁ and R₂ are independently a hydrogen atom, or an alkyl, acyl or alkoxy group of 1-30 carbon atoms, each block ranging in molecular weight from 0.1 to 500 kg/mol and preferably from 1 to 100 kg/mol. Preferably, the block copolymer is poly(ferrocenyldimethylsilane-b-isoprene).

In the present invention, the enzyme may be prepared, together with the self-assembling block copolymer, into a solution, and applied to an electrode to form a coating membrane. The coating membrane may preferably range in thickness from 1 to 50 µm while the enzyme is used in an amount of 1-50 wt % based on the total weight of the coating membrane, preferably in an amount of approximately 5-45 wt %, and most preferably in an amount of 30 wt %.

In accordance with an aspect thereof, the present invention provides a block copolymer, comprising a conductive organometallic block, which is self-assembled to an amorphous bicontinuous structure or a nanoparticle.

In accordance with another aspect thereof, the present invention provides a biofuel cell capable of generating a current using an electrode comprising a self-assembling block copolymer and an enzyme. The biofuel cell may utilize a sugar, for example, glucose, or an alcohol, as a fuel for electricity generation.

In accordance with a further aspect thereof, the present invention provides a biosensor, designed to measure a concentration of sugar, such as glucose, comprising an electrode composed of an organometallic block copolymer and an enzyme.

Also, the present invention is concerned with the electrochemical characteristic of an enzyme integrated into a nanostructured, self-assembled redox polymer.

In one preferred embodiment of the present invention, the enzyme is glucose oxidase (GOx) while an organometallic block copolymer with poly(ferrocenyldimethylsilane) is employed as an electron mediator. In this regard, the synthesis and self-assembly nature of ferrocene (Fc)-containing block copolymers are discovered. The Fc moieties packed within the self-assembled structures aim to increase the electron transfer rate between the flavin adenine dinucleotide (FAD) cofactor in GOx and an electrode surface. Upon synthesis, alterations in molecular weight and solvent composition offer various nanostructures of poly(ferrocenyldimethylsilane-b-isoprene) (PFDMS-b-PI) block copolymers, including bicontinuous structures, nanowires, and nanoparticles, which are, in turn, different in catalytic current activity. Osmium decoration for PI crosslinking increases the stability of the electrode stable within physiological environments.

In one embodiment of the present invention, a glucose biosensor may be fabricated with the electrode and may be applied to implantable bio-micro devices.

### Advantageous Effects

As described hitherto, the functional electrode which is fabricated using an enzyme and a nanostructured organometallic block copolymer in accordance with the present invention exhibits a high electron transfer rate, and is very stable within physiological environments because of the crosslinked structure of the copolymer. In addition, the present invention suggests a method for determining electrochemical characteristics of an enzymatic biofuel cell by modulating the morphology of a redox polymer-based electron mediator.

### Description of Drawings

FIG. 1 shows TEM images of structures of PFDMS-b-PI block copolymers in different solvent mixtures.
FIG. 2 shows FIB-TEM images of the GOx/PFS-PI_{OS} nanowires.
FIG. 3 shows SEM images of the PFS-PI_{OS} electrode.
FIG. 4 shows cyclic voltammetry measurements of electrodes as a function of glucose concentration, indicating the dependence of electron mediation on polymer structures.
FIG. 5 shows current responses of a glucose sensor with the injection of glucose in a physiological environment.
FIG. 6 shows a fabrication process of nanowire-based functional electrodes composed of GOx and PFDMS-b-PI mediators.

### Mode for Invention

### EXAMPLE

### Synthesis of 1,1'-Dimethylsilylferrocenophane Monomer

A ferrocene containing monomer, 1,1'-dimethylsilylferrocenophane monomer was synthesized by coupling lithiated-ferrocene with silane, as described by the document of Manners et al. The disclosure of which is hereby incorporated by reference in its entirety. The synthesized monomer was purified by repeated sublime/recrystallization processes under vacuum.

### Synthesis of PFDMS-b-PI

PFS-b-PI block copolymers with different molecular weights were synthesized by sequential anionic polymerization. The PI precursor synthesis was performed in purified benzene as a solvent and s-butyllithium as an initiator. After synthesizing the PI chains with targeted molecular weights, a pre-weighed amount of the purified 1,1'-dimethylsilylferrocenophane monomer was added to the reaction chamber in the glove box. The reaction chamber was then returned to a vacuum line and was thoroughly degassed. A small quantity of purified THF was distilled into the reaction chambers to speed up the polymerization of the 1,1'-dimethylsilylferrocenophane monomer. As the 1,1'-dimethylsilylferrocenophane monomer polymerized, the color of the reaction solution changed from red to brown. The polymerization proceeded for at least six hours and then was terminated with isopropanol. Upon termination, the color of the solution changed from brown to red. The PFS-b-PI copolymers were precipitated by using hexane and the polydispersity indices of the copolymers were measured to be 1.08.

### Electrode Preparation

Glucose oxidase from Aspergillus niger (∼200 units/mg) was purchased from Sigma-Aldrich. For use in a functional electrode composed of GOx and a block copolymer, a 60 µM GOx solution and homogeneous 1 wt% PFDMS-b-PI solutions in a mixture of different solvents were prepared. GOx was deposited on a porous carbon electrode by dropping. Immediately after deposition, the GOx-deposited electrode was drop coated with the PFS-PI solutions. After complete evaporation of solvents in vacuum oven, the electrode was exposed to OsO4 vapor for 3 hrs. Afterwards, the electrode was stored in PBS buffer solution before use.

### Electrochemical Experiment

Cyclic voltammetry (CV) measurements were recorded using an EG&G PAR 273A in a three-electrode cell with a platinum gauze as a counter electrode and Ag/AgCl as a reference electrode. All of the potential measurements were obtained based on the Ag/AgCl reference electrode. The working electrode was a porous carbon electrode fabricated according to the method of the present invention. All measurements were performed in phosphate buffered saline (PBS) (pH 7.4) at room temperature and ambient atmosphere. A control test was made with PBS alone to give non-characteristic, plain CV measurements. All electrochemical experiments were repeated with more than 20 electrodes to produce data at a representative scan rate of 20 mV/s. For reliable data, the measurements of the first cycle were discarded, and recordings of subsequent cycles were taken.

### Morphology Characterization

Surface morphologies of fabricated electrodes were determined by atomic force microscopy (AFM) in tapping mode. All measurements were made with a phase contrast value of 10o. Samples for transmission electron microscopy (TEM) experiments were prepared by drop-coating of 0.1wt% PFDMS-b-PI copolymers in various solvent mixtures. All imaging has been performed before OsO4 decoration due to the enough electron contrast between Fc domains and PI chain domains using a Zeiss LIBRA 200FE microscope operating at 200 kV equipped with a cold stage (-160 oC) and an Omega energy filter. Fe elemental mapping was obtained using energy filtered imaging with the three-window method at the Fe L edge of 709 eV energy loss. Focused ion beam etched TEM (cross-sectional FIB-TEM) samples were prepared with a FEI Strata 235 Dual Beam FIB system operated at 30 kV. For the FIB-TEM imaging, surface protection of samples was accomplished by exposure to RuO4 vapor for 10 hours. Imaging of etched samples was performed with a JEOL 3010 microscope operated at 200 kV.

### Conductivity Measurement

Conductivities of PFDMS-b-PI block copolymers were measured using AC impedance spectroscopy in a glove box. For through-plane conductivities, in-house built two platinum electrodes with sizes of 1.15 cm x 2 cm were used as working/counter electrodes. Data were collected using a 1260 Solatron impedance analyzer operating over a frequency range of 1-100,000 Hz.

### Characterization of PFDMS-b-PI Block Copolymers

Ferrocene (Fc)-containing block copolymers with Os-reactive diene groups, BFDMS-b-PI, were synthesized by sequential anionic polymerization. Two different PFDMS-b-PI block copolymers were measured to have respective molecular weights of 10.4-8.0 kg/mol and 69.0-92.0 kg/mol. The above-illustrated chemical formula shows a chemical structure for the PFDMS-b-PI block copolymers wherein x and y indicate the degrees of polymerization of each block. In the block copolymer, the Fc-containing PFDMS chain is responsible for redox response while the electrochemical characteristics of the Fc moiety are the same as that of a small Fc molecule.

FIGS. 1B to 1E are TEM images of PFDMS-b-PI block copolymers in different solvent mixtures. PFDMS-b-PI block copolymers are observed to have nanostructures which vary depending on molecular weights and mole fractions of mixed solvents. For example, small molecular weight PFDMS-b-PI (10.4-8.0 kg/mol) in toluene/hexane (20/80 vol.%) mixtures yields bicontinous structures with spacings of ca. 200 nm (FIGS. 1B and 1C). In FIG. 1C, the dark region in FIG. 1B was found to be an Fc-rich area, as measured by Fe elemental mapping. When THF/hexane (20/80 vol.%) mixture is employed, in contrast, distinctly different morphology of nanowires with the lengths of several micrometers is seen. The diameter of the nanowires measured 20 nm. Without theoretical limitations, this may be counted by solubility parameter. The parameter solubility of PFDMS (18.6 MPa½) were observed to be similar to those of toluene (18.3 MPa½) and THF(18.5 MPa½) while the solubility of PI (16.2 MPa½) was near that of hexane (14.9 MPa½). In addition, it was found that the crystalline characteristics of PFDMS are significantly affected by the choice of solvents (toluene and THF).

While keeping the THF/hexane (20/80 vol.%) mixtures, the use of large molecular weight PFDMS-b-PI (69.0-92.0 kg/mol) results in fairly monodisperse nanoparticles with a size of 60 nm. In addition, it was confirmed that a solution of THF and hexane with 25/75 vol % resulted in short and relatively non-uniform nanowires 40 nm in diameter. This implies that the nanoparticle morphology is stable only at the narrow window of THF/hexane compositions.

The nanostructured PFDMS-b-PI organometallic block polymers may be utilized to transfer electrons from redox reactions of GOx/glucose to electrodes.

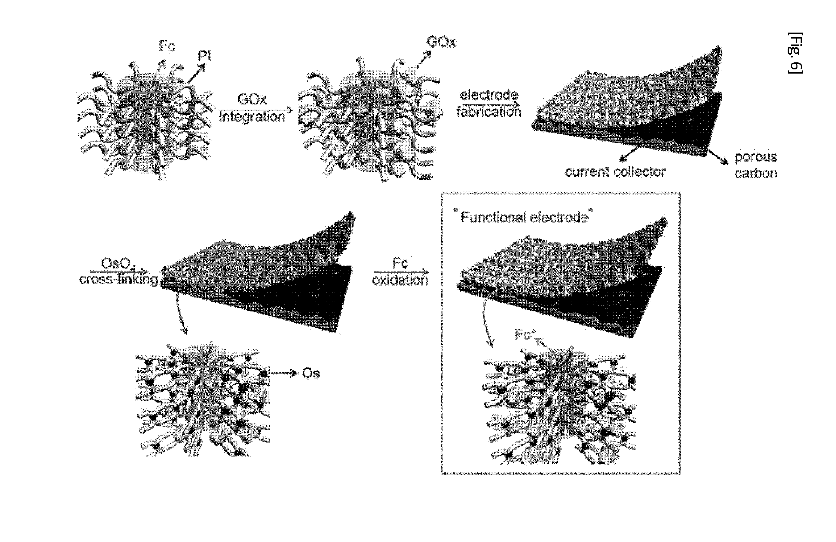

FIG. 6 shows a fabrication process of functional electrodes composed of GOx and PFDMS-b-PI mediators. For brevity, it is depicted based on the nanowire morphology of the copolymers. GOx is deposited on porous carbon electrode by dropping aliquots of 60 µM GOx stock solution until targeted mass is attained. Homogeneous 0.1 wt% PFS-PI solutions prepared by different solvent mixtures are then immediate drop-coating onto the GOx deposited electrode to a film thickness of 1 to 50 µm. The maximum catalytic current is observed for GOx concentration of 30 wt %. At very high enzyme contents above 50 wt %, precipitation of GOx was observed.

In the present invention, PI is used as a supporting matrix since the diene groups in PI chains allow for the introduction of cross-linking points. The chemical cross-linking has been carried out by exposing the electrodes to OsO4 vapor for 3 hrs in which the Os of OsO4 moieties forms covalent bonds with hydrocarbon groups in close proximity. Hereinafter, the mediator polymer thus fabricated is referred to as PFS-PIOS. After completion of Os staining, unfixed GOx is washed off with distilled water. It should be noted here that without Os decoration, the film becomes unstable within the physiological environments. As a final step, activation of the Fc moiety is carried out by applying anodic potential sweep. Because the Fc moieties will be positively charged after activation, it is expected to enhance association with the negatively charged GOx.

### Structural Analysis of Fabricated Functional Electrode

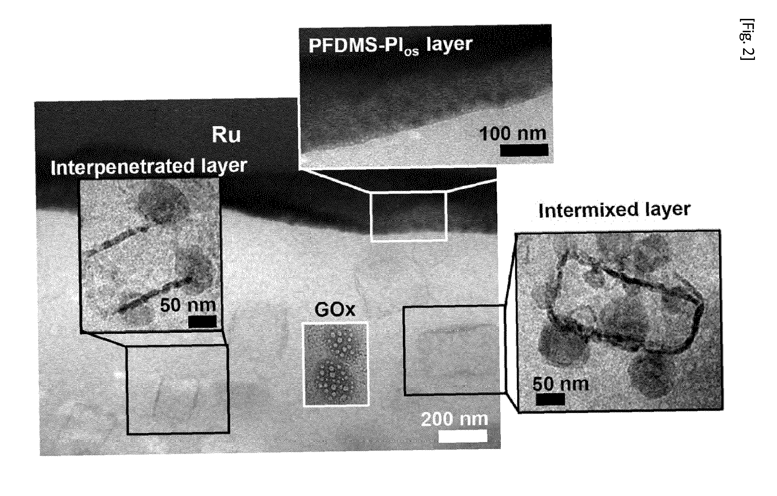

The structural analysis of the fabricated electrodes was observed by combining FIB, TEM, and SEM. FIG. 2 presents the FIB-TEM images of the nanowire electrode composed of GOx and PFDMS-b-PIOS. To minimize the sample damage caused by electron beams during a milling process, ruthenium (Ru) deposition on the surface of the electrode is performed before observation. As can be seen from FIG. 2, the electrode comprises layers of PFDMS-B-PIOS mediators and GOx with some intermixing and interpenetration of these materials. The thickness of the PFDMS-b-PIOS layer at the top surface of the electrode is approximately 100-200 nm. The cross-sectional view of nanowires with 20 nm diameter is shown in the upper right inset image. In the intermixed/interpenetrated layers, the GOx are detected as 40 ^{∼} 80 nm sized aggregates. It should be noted here that the GOx was extremely unstable under the electron beam even both in the presence of cold stage (-160ºC) and at a low dose rate (9.6 e-/Å2). Typical example of GOx degradation is shown inside the white inset box with different contrast.

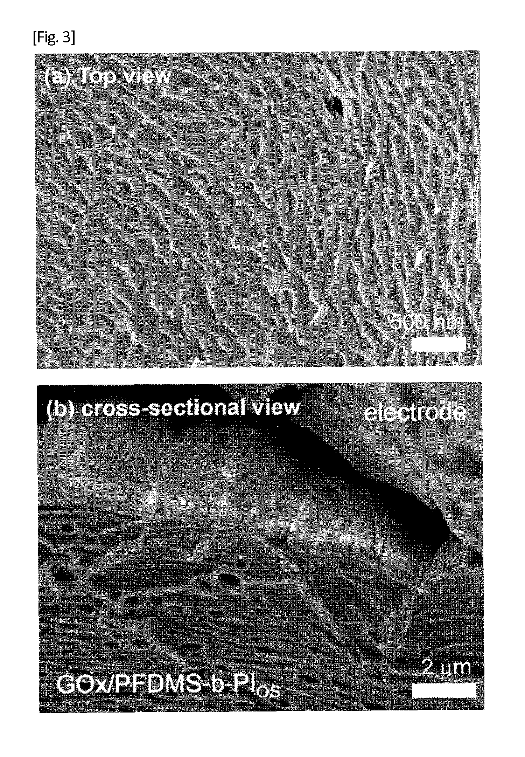

The structure of bicontinuous PFDMS-b-PIOS electrodes was investigated by scanning electron microscopy experiments. As shown in FIG. 3A, the top view of the fabricated electrode is well agreed with the image seen in FIG. 1B. Upon examining the cross-sectional structure of the electrode, it is found that the electrode is porous so that glucose can have access to the enzymes, yet it provides a protective cage for immobilizing the GOx without affecting biological function. The current collector is intentionally delaminated with an aid of liquid nitrogen to examine the topology of bottom side of the electrode. As can be seen in FIG. 3B, the bicontinous morphology was again revealed, which leads to the conclusion that PFDMS-b-PIOS mediators exist at both air surface and the substrate. However, it is difficult to clearly identify the structure of the electrode in the cross sectional FIB-TEM images because the size of bicontinous polymer (200 nm) is greater than the thickness of the electrode material (80-120 nm).

### Glucose Oxidation by Electrode Composed of GOx/ PFDMS-b-PIOS Mediator

To demonstrate the efficiency of immobilization and wiring of GOx into the electrode by the networks of PFS-PIOS mediators, cyclic voltammetry (CV) measurements of the fabricated electrode were first carried out. The concentration of the Fc moiety was fixed as 2 mM and a low scan rate of 20 mV was used. As shown in FIG. 3A, in the absence of GOx, the well-defined current responses upon electrochemical cycling of the Fc moieties of the Ag/AgCl reference electrode were detected at 420 mV and 550 mV. Taking into consideration the fact that the standard reduction potential of Fc is known as Eo = 400 mV, this change was regarded reasonable. When GOx was incorporated into the PFDMS-b-PIOS nanowires, however, distinctly difference redox responses were seen. New anodic and cathodic waves at 390 and -80 mV were detected while the oxidation peak at 420 mV disappeared. The shift of oxidation peak from 550 to 390 mV reflected the change in solvation of the Fc moieties due to the polar environment provided by GOx. The disappearance of Fc reduction peak at 420 mV implies that Fc+ is clearly used up in the catalytic regeneration of GO(FAD) given below:

GO(FADH2) + 2Fc+ → GO(FAD) + 2Fco + 2H+

where Fc regenerated in above catalytic cycle is again oxidized to Fc+ at the anode.

The standard reduction potential of FAD (vs SHE) is known to be -180 mV. Such characteristic change in electrochemical response of the electrode indicates that catalytically active GOx has been successfully embedded into the network of PFDMS-b-PIOS mediator, yielding the effective communication with Fc sites. Herein, no redox responses of osmium (Os(III)/Os(II)) were found, indicating that the osmium moiety is sensitive to local concentrations.

To explore the morphology effects on catalytic responses of the electrodes, CVs of electrodes with different morphologies were recorded a function of glucose concentration. The PFDMS-b-PIOS nanowire was utilized as an electron mediator, as shown in FIG. 3B. In this regard, CV measurements were conducted in glucose in PBS. As can be seen in FIG. 3B, even with 1.3 mM of glucose, 40% increment in peak currents was seen. With the increase in the amount of glucose, catalytic currents gradually increase until the values level off at 60 mM of glucose. In the inset figure, the cathodic current at -80 mV is plotted as a function of glucose concentration. The inset AFM image shows the surface topology of the fabricated electrode.

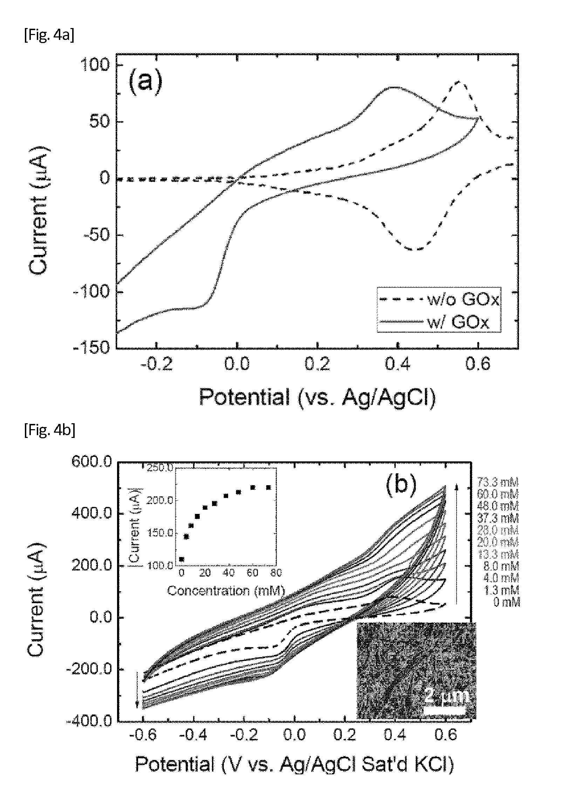

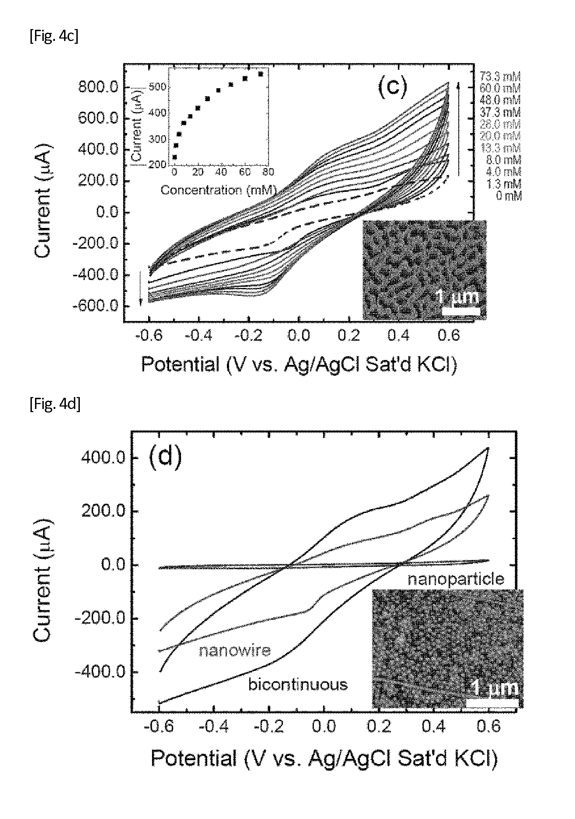

The redox process of the electrodes appears to greatly vary depending on the morphology of PFDMS-b-PIOS mediators. When the morphology of PFDMS-b-PIOS copolymer is switched to bicontinous phase, as shown in FIG. 4C, analogous redox waves to the case of nanowire are seen. However, an increase of almost two times the peak current is detected at the same level of glucose concentration and the current at-150 mV reaches high and stable value of 550 µA/cm2 at 60 mM glucose. Consequently, the enhanced catalytic responses with the use of bicontinous PFDMS-b-PIOS mediators can be rationalized by the better connectivity of the bicontinuous structure as well as larger contact area between PFS domains and entrapped GOx. It should be noted here that the electrodes were very stable regardless of morphologies of PFS-PIOS mediators so that less than 2% of the peak current was lost over 75 consecutive redox-cycles.

FIG. 4D summaries the morphology effects on the current density of GOx/PFDMS-b-PIOS electrodes. One representative set of CV data was plotted at glucose concentration of 8 mM, which is similar to the blood glucose level. Among nanowire, nanoparticle, and bicontinuous morphology; the GOx confined by bicontinuous PFS exhibits the best activity for the GOx/glucose redox reactions. This gives sufficient basis to the further leftward shift of the reduction peak of the bicontinuous morphology than that observed with the nanowire morphology. Interestingly, when the morphology effect was further exploited by employing nanoparticle-forming PFDMS-b-PIOS, fairly negligible redox activity is seen, signaling insufficient communication between Fc moieties and GOx due to the isolated spherical PFS domains. From the data obtained so far, there is no doubt about the importanct role of morphologies of the redox copolymers on determining the electron mediating ability of functional electrodes.

Cyclic voltametry measurements were recorded using the electrode fabricated only with the bicontinous PFDMS-b-PI_{OS} block copolymer. Given the same diameter, the bicontinous structure, when prepared from a PFDMS-b-PI_{OS} block copolymer with the same molecular weight, produces a current two times that of the nanowire structure (FIG. 4D). From this data, it is understood that the bicontinous PFDMS-b-PI_{OS} block copolymer is oxidized more than two times as efficiently as the nanowire thanks to its larger contact area. Quite different values of conductivity can be obtained according to the oxidation efficiency of iron. Through-plane conductivities of PFS-PI_{OS} copolymers with different morphologies were measured at room temperature to elucidate the morphology effects, which should be a key ingredient in modeling how electron transfer reactions couple to GOx and a substrate. Interestingly, the conductivities of activated PFS-PI_{OS} copolymers diverge as 1.34×10⁻⁵ (bicontinous) and 4.03×10⁻⁶ S/cm (nanowire).

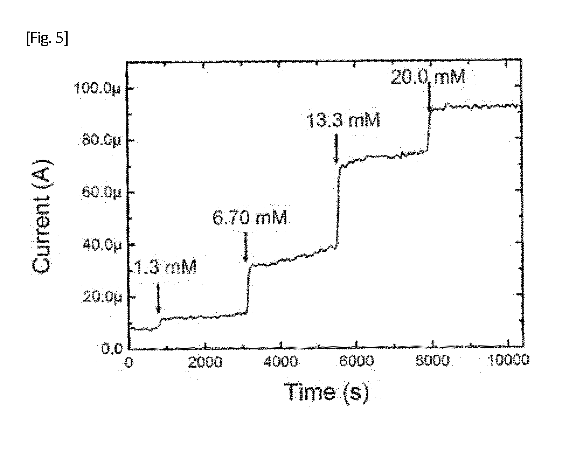

An examination was made of the bio-sensing ability of the electrodes according to the present invention. The biocatalytic activity of the electrodes was evaluated upon the injection of glucose with different concentrations to the three-electrode system. Because the physiological concentration of glucose in blood is between 5 and 10 mM, it is essential to obtain a sufficient electrical signal at around 10 mM of glucose. The electrode fabricated with the bicontinuous PFDMS- b-PI_{OS} block copolymer was operated very sensitively even at a very low amount of GOx (10 µL). The current responses at cathodic wave around -0.2 V vs. Ag/AgCl were recorded over time. As shown in FIG. 5, the addition of glucose to the physiological environments causes a step changes in the observed current with a final steady state value proportional to the concentration of glucose, as a result of continuous electron transfer from the enzyme to the Fc units. In particular, even with fairly small amount of glucose, 1.3 mM, a rapid and obvious current response was found as a discontinuous increase in peak current from 7 to 12 µA Approximately 35 min of equilibrium is allowed at each glucose concentration and the electrode exhibited excellent stability.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. An electrode, comprising a self-assembling organometallic block copolymer and an enzyme.

2. The electrode of claim 1, wherein the block copolymer is, at least in part, crosslinked.

3. The electrode of claim 1 or 2, wherein the block copolymer comprises a crosslinking block with a double bond.

4. The electrode of any one of claims 1 to 3, wherein the block copolymer is self-assembled with an organic metal to an amorphous bicontinous structure, a nanowire, and a nanoparticle.

5. The electrode of any one of claims 1 to 4, wherein the block copolymer is poly(ferrocenyldimethylsilane-b-isoprene).

6. The electrode of any one of claim 1 to 5, wherein the block copolymer, together with the enzyme, forms a coating layer with a thickness of 1-50 µm.

7. The electrode of claim 6, further comprising a porous carbon layer beneath the coating layer.

8. The electrode of claim 6 or 7, wherein the coating layer contains the enzyme in an amount of 1-50 wt% based on the total weight of the coating layer.

9. The electrode of any one of claims 6 to 8, wherein the coating layer contains the enzyme in an amount of 30 wt % based on the total weight of the coating layer.

10. An amorphously self-assembled block copolymer, comprising a block with a conductive organometal.

11. The amorphously self-assembled block copolymer, wherein the block is at least in part crosslinked with the organometal.

12. The amorphously self-assembled block copolymer, being represented by the following General Formula: 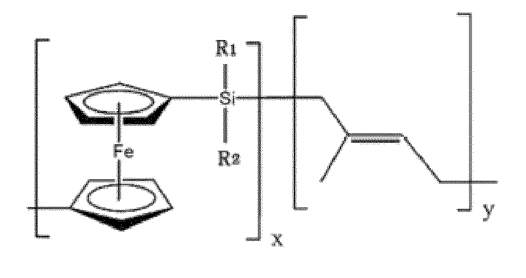 wherein
x and y indicate degrees of polymerization for respective blocks, and
R₁ and R₂ are independently a hydrogen atom, or an alkyl, acyl or alkoxy group of 1-30 carbon atoms, each block ranging in molecular weight from 0.1 to 500 kg/mol.

13. The amorphously self-assembled block copolymer of any one of claims 10 to 12, further comprising an enzyme generating an electron, said electron being transferred by the organic metal.

14. A biofuel cell, generating electricity using the electrode of any one of claims 1 to 9.

15. A biosensor, using the electrode of any one of claims 1 to 9.

16. The biosensor of claim 15, being a glucose sensor.

17. A method for fabricating a biosensor, comprising coating an electrode with a mixture of a self-assembling organometallic block copolymer and an enzyme.

18. The method of claim 18, wherein the self-assembling block copolymer is self assembled to an amorphous bicontinuous structure.

19. The method of claim 17 or 18, wherein the block copolymer is crosslinked.

20. The method of any one of claims 17 to 19, wherein the block copolymer is poly(ferrocenyldimethylsilane-b-isoprene).
